# EUROPEAN PATENT APPLICATION

(11) **EP 2 175 033 A1**
(43) Date of publication of application: **14.04.2010**
(21) Application number: 08710822.1
(22) Date of filing: 30.01.2008
(51) Int. Cl.: C12P 17/06, C07D 309/38

(54) **LARGE-SCALE PURIFICATION METHOD FOR 2-PYRONE-4,6-DICARBOXYLIC ACID**

(30) Priority: 08.08.2007 JP 2007206947
(71) Applicant: Kabushiki Kaisha Toyota Jidoshokki, Kariya-shi, Aichi 448-8671 (JP)
(72) Inventor: MASE, Kohei, Kariya-shi Aichi 448-8671 (JP); SHIMO, Toshihisa, Kariya-shi Aichi 448-8671 (JP); OHARA, Naoki, Kariya-shi Aichi 448-8671 (JP); KATAYAMA, Yoshihiro, Fuchu-shi Tokyo 183-8538 (JP); SHIGEHARA, Kiyotaka, Fuchu-shi Tokyo 183-8538 (JP); YAMAMOTO, Yusuke, Kariya-shi Aichi 448-8671 (JP); MURASE, Hitotoshi, Kariya-shi Aichi 448-8671 (JP)
(74) Representative: TBK-Patent
(86) International application number: PCT/JP2008/051890
(87) International publication number: WO 2009/019900

(57) **Abstract**

To provide an industrial purification method of PDC obtained by fermentative production.

A method of purifying 2-pyrone-4,6-dicarboxylic acid which comprises including a salt of monovalent to tetravalent cations in a fermentation liquid containing microbially-produced 2-pyrone-4,6-dicarboxylic acid; and a method of purifying 2-pyrone-4,6-dicarboxylic acid which method comprises extracting 2-pyrone-4,6-dicarboxylic acid from a fermentation liquid containing microbially-produced 2-pyrone-4,6-dicarboxylic acid without forming 2-pyrone-4,6-dicarboxylate.

## Description

### FIELD OF THE INVENTION

The present invention relates to an industrial purification method of 2-pyrone-4,6-dicarboxylic acid obtained by fermentative production.

### BACKGROUND ART

Lignin, a major component of plants, is a biomass resource ubiquitously contained as an aromatic polymer compound in the cell walls of plants. However, plant-derived aromatic compounds having lignin as a main component are composed of a variety of ingredients and/or have complicated polymer structures, and thus technologies for their useful utilization have not been developed. Methods of using them so far known include one in which a fragrance material, vanillin, is separated from a low molecular weight aromatic digest produced by chemical decomposition such as the alkali decomposition of said aromatic component. At present, however, no useful methods of utilization are known for large quantities of low molecular weight aromatic compounds other than vanillin produced by chemical decomposition. Thus, lignin produced in large quantities during the process of making paper has been burned as an alternative fuel to petroleum instead of being used usefully.

On the other hand, the present inventors have found that plant-derived fragrance aromatic compounds such as lignin can be converted to low molecular weight mixtures including vanillin, syringaldehyde, vanillic acid, syringic acid and protocatechuic acid, by chemical decomposition such as hydrolysis, oxidative decomposition and solvolysis, by physicochemical decomposition with a supercritical water or a supercritical organic solvent or the like, and these five compounds can be converted to 2-pyrone-4,6-dicarboxylic acid, a single intermediate of raw materials for functional plastics and chemical products.

The present inventors have also reported a method of producing 2-pyrone-4,6-dicarboxylic acid from these five compounds using transformed cells having a gene encoding four types of enzymes (benzaldehyde dehydrogenase, demethylase, protocatechuic acid 4,5-dioxygenase, 4-carboxy-2-hydroxymuconate-6-semialdehyde dehydrogenase) that are involved in a multistage process for the fermetative production of 2-pyrone-4,6-dicarboxylic acid (see, for example, Japanese Unexamined Patent Publication (Kokai) No. 2005-278549).

However, Japanese Unexamined Patent Publication (Kokai) No. 2005-278549 only describes an activated charcoal treatment as a purification method of 2-pyrone-4,6-dicarboxylic acid, and does not reveal the details. Also, although Japanese Unexamined Patent Publication (Kokai) No. 2000-32988 discloses a method of producing 2-pyrone-4,6-dicarboxylic acid in the presence of α-hydroxy-γ-carboxymuconate-s-semialdehyde, it makes no mention of the purification method.

### DISCLOSURE OF THE INVENTION

The problem to be resolved by the present invention is to provide an industrial purification method for fermentatively produced 2-pyrone-4,6-dicarboxylic acid that is useful as a raw material for functional plastics and a raw material for chemical products.

After intensive and extensive research considering such circumstances, the present inventors have found that by including a specific salt in a microbial fermentation liquid containing 2-pyrone-4,6-dicarboxylic acid, the corresponding 2-pyrone-4,6-dicarboxylate can be isolated at high purity and high yield, and therefore have completed the present invention. Also the present inventors have found that by extracting said fermentation liquid with a specific solvent without forming 2-pyrone-4,6-dicarboxylate, 2-pyrone-4,6-dicarboxylic acid can be isolated at high purity and high yield, and therefore have completed the present invention.

Thus,
(1) the present invention provides a method of purifying 2-pyrone-4,6-dicarboxylic acid, said method comprises including a salt of a cation selected from a monovalent, a divalent, trivalent and tetravalent cation in a fermentation liquid containing microbially-produced 2-pyrone-4,6-dicarboxylic acid.
(2) The present invention provides the purification method according to (1), wherein said monovalent, divalent, trivalent or tetravalent cation is a metal ion, an ammonium ion or an alkylammonium ion.
(3) The present invention provides the purification method according to (1) or 2, wherein said monovalent cation is selected from sodium, potassium, rubidium, silver, lithium and cesium.
(4) The present invention provides the purification method according to (1) or 2, wherein said divalent cation is selected from magnesium, calcium, iron (II), copper (II), zinc, barium, cobalt, nickel (II), manganese and chromium (II).
(5) The present invention provides the purification method according to (1) or 2, wherein said trivalent cation is selected from iron (III), aluminum and gallium.
(6) The present invention provides the purification method according to (1) or 2, wherein said tetravalent cation is selected from tin (IV), lead (IV), titanium (IV) and germanium (IV).
(7) The present invention provides the purification method according to any one of (1) to (6), wherein said salt of a monovalent, divalent, trivalent or tetravalent cation is a simple salt selected from a chloride, a bromide, a sulfate, a phosphate and a carbonate; a cyano complex; or a complex salt.
(8) The present invention provides the purification method according to (7), wherein said complex salt is a complex salt containing two types of cations.
(9) The present invention provides the purification method according to (7), wherein said salt of a monovalent cation is sodium chloride, potassium chloride, rubidium chloride, silver chloride, sodium bromide, sodium sulfate, disodium phosphate or dipotassium hydrogen phosphate.
(10) The present invention provides the purification method according to (7), wherein said salt of a divalent cation is magnesium chloride, copper sulfate, or potassium hexacyanoferrate (II).
(11) The present invention provides the purification method according to (7), wherein said salt of a trivalent cation is ferric chloride (III) or potassium hexacyanoferrate (III).
(12) The present invention provides the purification method according to (7) or (8), wherein said salt of a tetravalent cation is potassium stannate (IV).
(13) The present invention provides the purification method according to any one of (1) to (12), comprising the steps of collecting 2-pyrone-4,6-dicarboxylate deposited by the presence of said salt of a cation, dissolving it in water, and then extracting it with ethyl acetate, cyclopentanone or cyclohexanone under an acidic condition.
(14) The present invention provides the purification method according to (13), which comprises adding an excess amount of a strong acid at the above step of extraction.
(15) The present invention provides the purification method according to any one of (1) to (14), comprising the steps of collecting 2-pyrone-4,6-dicarboxylate deposited by the presence of said salt of a cation, dissolving it in water, and then treating said solution with a cation exchange resin.
(16) The present invention provides the purification method according to any one of (1) to (15), wherein at least two mole parts of said salt of a cation is used relative to 2-pyrone-4,6-dicarboxylic acid in said fermentation liquid.
(17) The present invention provides a method of purifying 2-pyrone-4,6-dicarboxylic acid which method comprises extracting 2-pyrone-4,6-dicarboxylic acid from the fermentation liquid containing microbially-produced 2-pyrone-4,6-dicarboxylic acid without forming 2-pyrone-4,6-dicarboxylate.
(18) The present invention provides the purification method according to (17), wherein said extraction is conducted with ethyl acetate, cyclopentanone or cyclohexanone.
(19) The present invention provides the purification method according to (17) or (18), wherein said extraction is conducted with ethyl acetate.
(20) The present invention provides the purification method according to any one of (17) to (19), wherein an excess of a strong acid is added at said extraction step.
(21) The present invention provides the purification method according to (20), wherein at least 3% by weight of said strong acid is used relative to said fermentation liquid.

### BRIEF EXPLANATION OF THE DRAWINGS

Fig. 1 is a drawing that shows the structure of a PDC-Na⁺ complex salt.
Fig. 2 is a drawing that shows the solubility in water of various PDC complex salts.

In accordance with the present invention, 2-pyrone-4,6-dicarboxylic acid can be produced in large quantities at high purity and high yield as well as at low cost from a microbial fermentation liquid containing 2-prone-4,6-dicarboxylic acid.

### BEST MODE FOR CARRYING OUT THE INVENTION

The PDC purification method of the present invention comprises obtaining PDC as a salt by including a salt of a monovalent, divalent, trivalent or tetravalent cation in a fermentation liquid containing microbially-produced 2-pyrone-4,6-dicarboxylic acid (hereinafter referred to as "PDC"), or as a free compound by preventing the formation of a PDC salt in said fermentation liquid.

The fermentation liquid containing microbially-produced PDC is not specifically limited as long as it is obtained by culturing a transformed cell containing a gene encoding an enzyme suitable for a multi- or one-step fermentative production of PDC in the presence of a plant-derived low molecular weight compound such as vanillin, syringaldehyde, vanillic acid, syringic acid and protocatechuic acid or a mixture thereof. As such a fermentation liquid, there can be mentioned, for example, one obtained by a method described in Japanese Unexamined Patent Publication (Kokai) No. 2005-278549. In the method described therein, usually 10-20 g of PDC per liter of the fermentation liquid is produced. Before the purification of PDC described below, it is preferred, that cells have been removed by centrifugation, adsorption to activated charcoal and the like. Adsorption to activated charcoal may preferably be conducted by adding activated charcoal to said fermentation liquid, mixing and stirring, and then removing the activated charcoal by filtration, etc., or by passing aid fermentation liquid through an activated charcoal-filled layer.

### <Method of forming a PDC salt>

To a PDC-containing microbial fermentation liquid, a salt of a monovalent, divalent, trivalent or tetravalent cation is included so as to deposit the corresponding salt of PDC.

As a monovalent, divalent, trivalent or tetravalent cation, there can be mentioned a monovalent, divalent, trivalent or tetravalent metal ion; an ammonium ion; and an alkylammonium ion such as an hexamethylenediamine, ethylenediamine, diethanolamine and triethanolamine.

As a monovalent cation, there can be mentioned, for example, a metal ion such as sodium, potassium, rubidium, silver, lithium and cesium. Among them, a sodium ion, a potassium ion, a rubidium ion, or a silver ion may be preferred.

As a divalent cation, there can be mentioned, for example, a metal ion such as magnesium, calcium, iron (II), copper (II), zinc, barium, cobalt, nickel (II), manganese and chromium (II).

As a trivalent cation, there can be mentioned, for example, an ion of iron (III), aluminum, potassium and the like.

As a tetravalent cation, there can be mentioned an ion of tin (IV), lead (IV), titanium (IV), germanium (IV) and the like.

As a salt of a monovalent, divalent, trivalent or tetravalent cation, there can be mentioned a simple salt such as a chloride, a bromide, a hydroxide, a nitrate, a sulfate, a phosphate, a carbonate, an acetate, an oxalate, a citrate, a tartrate, a fumarate, a maleate, a malate, a cyanate, and a thiocyanate; an ammine complex (for example, a copper tetraammine), a cyano complex (for example, a hexacyanoferrate complex), a halogeno complex (for example, a tetrachloroferrate complex), a hydroxy complex (for example, an alumine complex); a double salt (preferably a double salt containing two types of cations), and the like. As a salt of a monovalent cation, specifically there can be mentioned potassium chloride, rubidium chloride, silver chloride, sodium bromide, sodium sulfate, disodium phosphate or dipotassium hydrogen phosphate, with sodium chloride, potassium chloride, rubidium chloride, silver chloride or sodium bromide being preferred. As a salt of a divalent cation, specifically there can be mentioned magnesium chloride, copper sulfate or potassium hexacyanoferrate (II). As a salt of a trivalent cation, specifically there can be mentioned ferric chloride (III), potassium hexacyanoferrate (III), or an alum (for example, potassium alum, iron alum, ammonium iron alum). As a salt of a tetravalent cation, specifically there can be mentioned potassium stannate (IV).

A mixture of two or more of these monovalent, divalent, trivalent or tetravalent cations may be used.

In addition to the above monovalent, divalent, trivalent or tetravalent cations, there can be used a vanadyl ion (VO²⁺), a titanate (TiO₂) ion, a cyanide ion, a thiocyan ion, an ionic compound containing thiocarbonyl and the like.

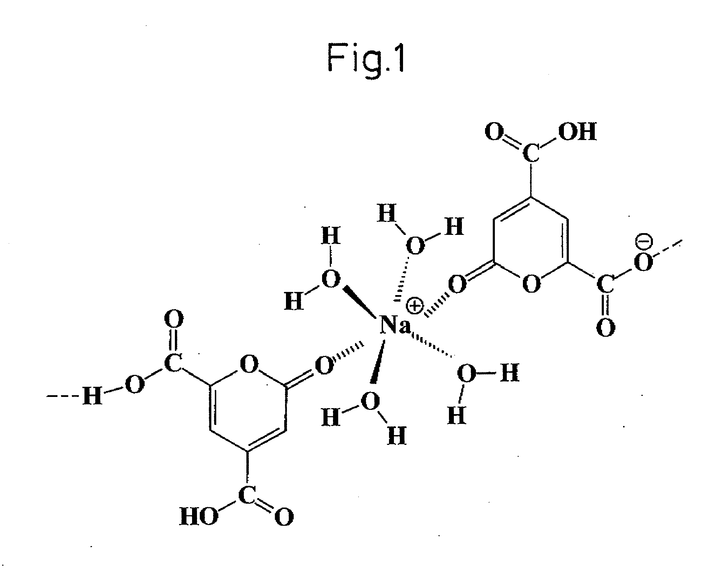

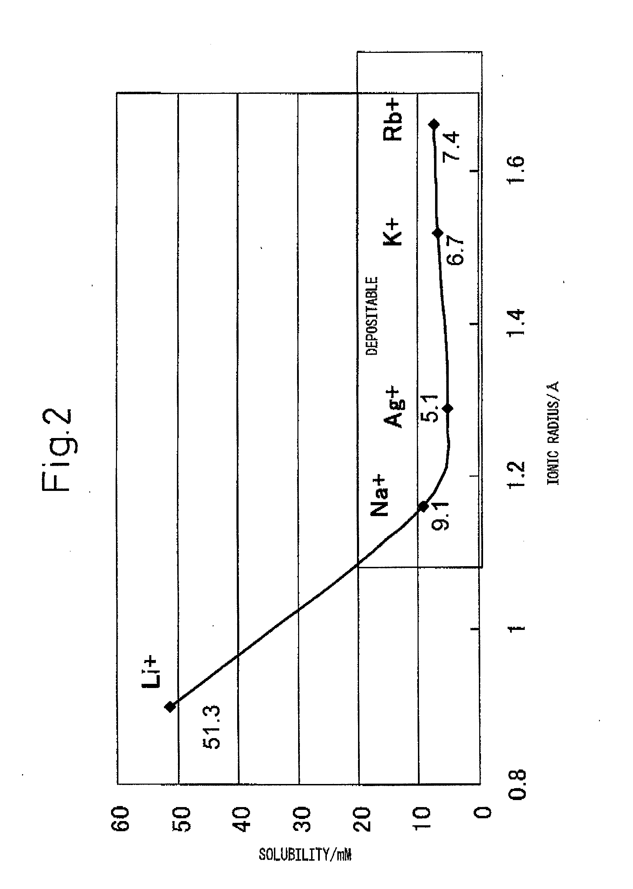

While PDC has a carboxylic group at positions 2 and 4, these carboxylic groups do not form a simple carboxylate of PDC by contacting with a salt of the above cation, but instead, as shown in Fig. 1, two PDC molecules form a hydrogen bond and thereby a complex salt assuming an octahedron 6-coordinate structure in which two carbonyl groups are coordinated surrounding a metal ion (a sodium ion in Fig. 1). Hereinafter, a complex salt formed centering on the carbonyl group of PDC may be referred to as "a PDC complex salt," in order to distinguish from a complex salt described in the section of salts of cations. The structure of a PCD complex salt is described in many references (for example, Acta. Cryst. (1992) C48, 460-465). Fig. 2 shows the water solubility of a PDC complex salt formed with PDC and a monovalent cation. As can be seen from Fig. 2, among the complex salts formed with PDC and a monovalent cation, the sodium salt, potassium salt, rubidium salt or silver salt of PDC was found to have a very low water solubility as compared to free PDC, components of microbial culture media, water-soluble components extracted from various plants (the water solubility of free PDC: 182 mM). Thus, it was demonstrated that the sodium salt, potassium salt, rubidium salt or silver salt of PDC is most suitable for the isolation of PDC since it can be easily separated from many of the culture medium components and water-soluble components of plant extracts and can be deposited.

Salts of the above cations may preferably be used in a large excess relative to PDC present in the microbial fermentation liquid, and for example, at least 2 mole parts, specifically 2-10 mole parts may preferably be used. In order to increase the salting-out effect, the fermentation liquid having said salt of a cation may be cooled or concentrated. When it is cooled, said fermentation liquid may be allowed to stand at 0-4°C for 12-24 hours. The PDC complex salt salted out may be collected by filtration.

The above PDC complex salt may be obtained as a futther highly pure free PDC by further conducting the following two different purification steps.
(I) The PDC complex salt can be extracted as free PDC by dissolving the above PDC complex salt in water, for example, pure water, and extracting it with an organic solvent under an acidic condition (about pH 1-2). As an extraction solvent, there can be preferably mentioned, for example, ethyl acetate, cyclohexanone, cyclopentanone, hexane, heptane, toluene, benzene, diethylether, tetrahydrofuran, chloroform and dichloromethane, with ethyl acetate, cyclohexanone or cyclopentanone being preferred, and ethyl acetate most preferred due to its low boiling point. In order to make an acidic condition, an excess amount, preferably 3% or more, more preferably 3% to 7%, of an aqueous strong acid solution relative to the aqueous layer may be used. As a strong acid, there can be mentioned hydrochloric acid, sulfuric acid, phosphoric acid, nitric acid, etc., with hydrochloric acid specifically preferred. If needed, the organic layer obtained may be treated for few times with an aqueous strong acid solution. The purity of the PDC obtained may be further enhanced, as needed, by repeating recrystallization.
(II) A free PDC can also be obtained by dissolving the above PDC complex salt in water, for example, pure water, and treating the solution with a H-type cation exchange resin. As a H-type cation exchange resin, there can be used, for example, Amberlite^{™} IR120B, DIAION^{™} SK1B, etc., with an ion exchange volume of about 2.0 meq/ml that are gel-type polystyrene/sulfonic acid type ion exchange resin.

From the solvent concentrate of PDC or the effluent concentrate of the ion exchange resin, PDC can be obtained as crystals by recrystallization. By repeated recrystallization, as needed, the purity of the crystals can be further enhanced. The present purification method that forms a PDC complex salt does not' require the extraction procedure of a large volume of a microbial fermentation liquid, can easily purify PDC as a salt by salting out, and, at the same time, can easily remove organic molecules having water solubility higher than the of a PDC complex salt.

### <Method of avoiding the formation of a PDC complex salt>

By extracting a PDC-containing microbial fermentation liquid with an organic solvent under an acidic condition, PDC can be isolated as a free PDC compound. As an organic solvent used in the present invention, there can be mentioned, for example, ethyl acetate, cyclohexanone, cyclopentanone, hexane, heptane, toluene, benzene, diethylether, tetrahydrofuran, chloroform and dichloromethane, with ethyl acetate, cyclopentanone or cyclohexanone being preferred, and ethyl acetate most preferred due to its low boiling point. Before the extraction with the above organic solvent, it is preferred to render said fermentation liquid highly acidic and to wash the post-extraction organic solvent with an aqueous strong acid solution, in order to prevent the extraction of a PDC complex salt. As a strong acid, there can be mentioned hydrochloric acid, sulfuric acid, phosphoric acid, nitric acid, etc., with hydrochloric acid specifically preferred. The amount combined of the strong acid to be used before and after the extraction with an organic solvent is preferably 3-7% relative to the amount of the microbial fermentation liquid and most preferably 3%-5%.

In order to remove protein in the above fermentation liquid, a relatively low boiling polar solvent such as acetone and an alcohol such as methanol and ethanol may be included in said fermentation liquid before the above extraction step. When alcohols are included, the acid may preferably be added after the removal of the alcohol.

By concentrating the above organic layer under reduced pressure in accordance with a conventional method and recrystallizing the concentrated dry sold in pure water, PDC can be obtained as crystals. By repeating recrystallization, the purity of the PDC crystals can be further enhanced.

According to the present purification method that does not include the formation of a PDC complex salt, PDC can be obtained as a completely free compound at a high PDC purity (99.5% to 99.9%) at one extraction procedure. Since the PDC obtained by the present method does not include its salt, it can be easily dissolved in various solvents, and a wide application can be expected as synthetic materials, etc., for polymers and chemical products.

### EXAMPLES

The present invention will be explained in detail with reference to specific examples, but it should be noted that the present invention is not limited to these examples in any way.

### (Materials)

A microbial fermentation liquid containing PDC was obtained by a method described in Japanese Unexamined Patent Publication (Kokai) No. 2005-278549.

### Example 1: A purification method for forming a PDC sodium salt

A microbial fermentation liquid (1.5 L) containing PDC was centrifuged (4000 rpm, 1 hour, 4°C) to remove the cells. To the supernatant obtained, 15 g of sodium chloride was added and allowed to stand at 4°C for 12 hours to deposit a PDC sodium salt. By collecting this by filtration, a crude PDC sodium salt was obtained (22 g).

Subsequently, 10 g of this crude PDC sodium salt was dissolved in 600 ml of pure water, adjusted to pH 1.5 with 3N HCl, and extracted with ethyl acetate (100 ml x three times). The ethyl acetate layer was washed in 100 ml of hydrochloric acid (pH 1.0), concentrated under reduced pressure, and crystallized at 4°C. The crystals were collected by filtration, dissolved in pure water, recrystallized, and dried under reduced pressure to obtain 5.7 g of PDC (recovery 70%). The purity was determined by HPLC to be 98.5%.

HPLC analytical condition: Instrument: Waters; flow rate: 0.2 ml/min; injection: 50 µl; column: 4.6φ x 250 mm (Senshu Scientific Co., Ltd, ODS-125I-SS); mobile phase: water:acetonitrile:acetic acid = 74:25:1; detection wavelength: 294 nm.

### Examples 2-11

To 25 ml of pure water, 0.25 g (1.4 mmol) of PDC (free compound) was added to obtain an aqueous PDC solution (0.054 mol/L). Then, to this aqueous PDC solution, a metal salt described in Table 1 below was added at an amount described. Depending on the type of the metal salt, some deposited immediately, some deposited after heating and cooling, and some deposited after concentrating water. After the deposit (or precipitate) was filtered, it was dried overnight at 60°C, and the deposition rate (recovery) of PDC was calculated from the weight of the deposit based on the reported composition (Na₂PDC₂H₂O) of the deposit with sodium chloride. The result is shown in Table 1.

**Table 1**

| Ex. | Metal salt | Amount added (g) | Solubility | Deposition condition | Weight deposited (g) | Color of deposit | Amount of PDC deposited (g) | Deposition Rate of PDC (%) |
|---|---|---|---|---|---|---|---|---|
| Ex.2 | NaBr | 0.4198 | Soluble (heat) | ↓ | 0.120 | White | 0.382 | 38.2 |
| Ex.3 | Na₂SO₄(anhydrous) | 0.2894 | Soluble | ↓ | 0.040 | White | 0.127 | 12.7 |
| Ex.4 | Na₂PO₄12H₂O | 0.2874 | Soluble (heat) | ↓ | 0.020 | White | 0.064 | 6.4 |
| Ex.5 | K₂HPO₄ | 0.3546 | Soluble | Concentration | Trace | | - | - |
| Ex.6 | MgCl₂6H₂O | 0.2507 | Soluble | Concentration | Trace | White | - | - |
| Ex.7 | CuSO₄5H₂O | 1.0170 | Soluble | Cooling | Trace | Light blue | - | - |
| Ex.8 | FeCl₃6H₂O | 1.1022 | Soluble | ↓ (Coagulation) | 0.060 | Reddish brown | 0.045 | 4.5 |
| Ex.9 | K₃[Fe(CN)₆] | 0.4632 | Soluble | ↓ | 0.050 | Blue | 0.038 | 3.8 |
| Ex.10 | K₄[Fe(CN)₆]3H₂O | 0.4422 | Soluble | ↓ | 0.140 | Blue | 0.108 | 10.9 |
| Ex.11 | K₂Sn(OH)₆ | 0.2306 | Soluble | ↓ | 0.263 | Milky-white | 0.693 | 69.3 |

| | | | | | | | | |
|---|---|---|---|---|---|---|---|---|
| ↓: Deposited immediately after the addition of a metal salt. | | | | | | | | |

### Example 12: A purification method that comprises the formation of a PDC sodium salt

A crude PDC sodium salt (10 g) obtained as in Working Example 1 was dissolved in 600 ml of pure water, which was passed through a column (40 mµφ) prepared from 100 g of a H-type ion exchange resin (Amberlite) with an ion exchange volume of 2.2 meq/ml. This was washed with pure water, and then developed with hydrochloric acid of pH 1 to collect the effluent fraction, concentrated under reduced pressure, and crystallized at 4°C. The crystals were collected by filtration, dissolved in pure water, recrystallized, and dried at 60°C under reduced pressure to obtain 7.2 g of PDC (recovery 88%, purity 98.5%).

### Example 13: A purification method of avoiding the formation of a PDC sodium salt

To 1.5 L of the PDC stock solution (a filtrate after microbial culture), 1.5 L of acetone and 45 ml of concentrated hydrochloric acid were added, stirred and mixed. To the mixture, 20 g of activated charcoal (037-02115, Wako Pure Chemical Industries, Ltd.) was added, stirred for 15 minutes, and then Celite (08003-02, Celite 503, Kanto Chemical Co. Inc.) was spread on a #131 filter paper, and suction filtered to remove the activated charcoal. From the filtrate, acetone was evaporated under reduced pressure to a liquid volume of 1.5 L. To the concentrate, 18 ml of concentrated hydrochloric acid was added, and extracted three time with 230 ml of ethyl acetate plus twice with 150 ml ethyl acetate. To the ethyl acetate extract, 36 g of anhydrous magnesium sulfate was added, dried, suction filtered, and evaporated to dryness under reduced pressure. The solid obtained was dried under reduced pressure (55°C) for 3 hours, dissolved in 15 ml of distilled water at 60°C, to which 2 ml of concentrated hydrochloric acid was added, and allowed to stand in a refrigerator for recrystallization. The solid was collected by filtration, dried under reduced pressure (55°C) to obtain about 13.2 g of PDC (recovery 68%, purity 98.5%).

Also, by scaling up (PDC stock solution 50 L) the amount of the PDC stock solution, a similar recovery was obtained.

### Industrial Applicability

The present invention can be used as an industrial purification method for 2-pyrone-4,6-dicarboxylic acid from a microbial fermentation liquid containing 2-pyrone-4,6-dicarboxylic acid.

## Claims

1. A method of purifying 2-pyrone-4,6-dicarboxylic acid said method comprises: including a salt of a cation selected from a monovalent, a divalent, trivalent and tetravalent cation in a fermentation liquid containing microbially-produced 2-pyrone-4,6-dicarboxylic acid.

2. The purification method according to claim 1, wherein said monovalent, divalent, trivalent or tetravalent cation is a metal ion, an ammonium ion or an alkylammonium ion.

3. The purification method according to claim 1 or 2, wherein said monovalent cation is selected from sodium, potassium, rubidium, silver, lithium and cesium.

4. The purification method according to claim 1 or 2, wherein said divalent cation is selected from magnesium, calcium, iron (II), copper (II), zinc, barium, cobalt, nickel (II), manganese and chromium (II).

5. The purification method according to claim 1 or 2, wherein said trivalent cation is selected from iron (III), aluminum and gallium.

6. The purification method according to claim 1 or 2, wherein said tetravalent cation is selected from tin (IV), lead (IV), titanium (IV) and germanium (IV).

7. The purification method according to any one of claims 1 to 6, wherein said salt of a monovalent, divalent, trivalent or tetravalent cation is a simple salt selected from a chloride, a bromide, a sulfate, a phosphate and a carbonate; a cyano complex; or a complex salt.

8. The purification method according to claim 7, wherein said complex salt is a complex salt containing two types of cations.

9. The purification method according to claim 7, wherein said salt of a monovalent cation is sodium chloride, potassium chloride, rubidium chloride, silver chloride, sodium bromide, sodium sulfate, disodium phosphate or dipotassium hydrogen phosphate.

10. The purification method according to claim 7, wherein said salt of a divalent cation is magnesium chloride, copper sulfate, or potassium hexacyanoferrate (II).

11. The purification method according to claim 7, wherein said salt of a trivalent cation is ferric chloride (III) or potassium hexacyanoferrate (III).

12. The purification method according to claim 7 or 8, wherein said salt of a tetravalent cation is potassium stannate (IV).

13. The purification method according to any one of claims 1 to 12, comprising the steps of collecting 2-pyrone-4,6-dicarboxylate deposited by the presence of said salt of a cation, dissolving it in water, and then extracting it with ethyl acetate, cyclopentanone or cyclohexanone under an acidic condition.

14. The purification method according to claim 13, which comprises adding an excess amount of a strong acid at the above step of extraction.

15. The purification method according to any one of claims 1 to 14, comprising the steps of collecting 2-pyrone-4,6-dicarboxylate deposited by the presence of said salt of a cation, dissolving it in water, and then treating said solution with a cation exchange resin.

16. The purification method according to any one of claims 1 to 15, wherein at least two mole parts of said salt of a cation is used relative to 2-pyrone-4,6-dicarboxylic acid in said fermentation liquid.

17. A method of purifying 2-pyrone-4,6-dicarboxylic acid which method comprises extracting 2-pyrone-4,6-dicarboxylic acid from the fermentation liquid containing microbially-produced 2-pyrone-4,6-dicarboxylic acid without forming 2-pyrone-4,6-dicarboxylate.

18. The purification method according to claim 17, wherein said extraction is conducted with ethyl acetate, cyclopentanone or cyclohexanone.

19. The purification method according to claim 17 or 18, wherein said extraction is conducted with ethyl acetate.

20. The purification method according to any one of claims 17 to 19, wherein an excess of a strong acid is added at said extraction step.

21. The purification method according to claim 20, wherein at least 3% by weight of said strong acid is used relative to said fermentation liquid.
